# EUROPÄISCHE PATENTSCHRIFT

(11) **EP 3 773 258 B1**
(45) Veröffentlichungstag und Bekanntmachung des Hinweises auf die Patenterteilung: **15.09.2021**
(21) Anmeldenummer: 20705185.5
(22) Anmeldetag: 13.02.2020
(51) Int. Cl.: A61B 17/15

(54) **FIXIERUNGSKLAMMER UND AUSRICHTUNGSVORRICHTUNG**
FIXING CLAMP AND ALIGNING DEVICE
PINCE DE BLOCAGE ET DISPOSITIF D'ORIENTATION

(30) Priorität: 15.02.2019 DE 102019103880
(43) Veröffentlichungstag der Anmeldung: 17.02.2021
(73) Patentinhaber: Aesculap AG, 78532 Tuttlingen (DE)
(72) Erfinder: BÖTTINGER, Roland, 78604 Rietheim-Weilheim (DE); MEIER, Tim, 45147 Essen (DE)
(74) Vertreter: Winter, Brandl - Partnerschaft mbB
(86) Internationale Anmeldenummer: PCT/EP2020/053740
(87) Internationale Veröffentlichungsnummer: WO 2020/165332

(56) Entgegenhaltungen:
- US-B1- 6 221 035

## Beschreibung

### Technisches Gebiet

Die vorliegende Offenbarung betrifft eine Fixierungsklammer / Fixierungsklemme für eine Ausrichtungsvorrichtung, für insbesondere eine tibiale Resektion, zum fixierenden Klemmen einer Körperextremität eines Patienten, wie etwa einer Tibia bzw. einer Malleole eines Schienbeins, eines Knöchels, eines Beins oder eines Sprunggelenks, mit einem ersten Klammerarm und einem zweiten Klammerarm und mit einer Lagergabel oder Lagermulde für die Körperextremität mit einem ersten und einem zweiten Lagerschenkel, welche einen zentralen Montage- oder Anbindungsabschnitt zur Montage oder Anbindung an einen Einstellstab sowie ein erstes Drehgelenk und ein zweites Drehgelenk an der Lagergabel aufweist, an denen jeweils der entsprechend erste Klammerarm um eine erste Drehachse und der zweite Klammerarm um eine zweite Drehachse drehbar angelenkt bzw. angebunden ist. Daneben betrifft die Erfindung eine Ausrichtungsvorrichtung / Einstellführung / Extramedulläres Tibia Ausrichtinstrument (ETA) für eine tibiale Resektionsführung gemäß dem Oberbegriff des nebengeordneten Anspruchs.

### Hintergrund der Erfindung

Eine präzise Resektion von Knochen eines Patienten, insbesondere der Tibia, hat eine große Bedeutung für einen Erfolg einer Operation zur Implantation einer Gelenksprothese. Die Ebene der Resektion muss hierbei exakt lokalisiert werden, um einerseits ein Maß einer Knochenentnahme möglichst geringzuhalten, wobei andererseits gleichzeitig gewährleistet werden muss, dass auch das gesamte defekte Knochengewebe entfernt wird. Der Abgleich der Ebene in Bezug zu einer anatomischen Achse, insbesondere einer Tibia-Achse, muss bei einer Operation kontinuierlich kontrolliert werden, um die Ausrichtung der Gelenksoberflächen des Gelenks über den gesamten Bereich der Gelenksbewegung zu gewährleisten.

Die exakte Festlegung einer tibialen Resektionsebene in einem Kniegelenk wird üblicherweise unter Verwendung einer Ausrichtungsvorrichtung bzw. einer Einstellführung (für einen Sägeblock) mit einem säulenförmigen Einstellstab eingestellt, der entfernt zur Tibia nahe am Fußknöchel befestigt wird. Der Einstellstab erstreckt sich dabei entlang der Tibia (im Wesentlichen) parallel zu der zugehörigen anatomischen Tibia-Achse. Die Resektionsebene kann dann in Bezug auf die Tibia-Achse definiert werden. Ein an der Ausrichtungsvorrichtung befestigter Säge- bzw. Führungsblock (Schneidblock / Führungsvorrichtung) definiert schließlich die Ebene der Resektion. Üblicherweise weist der Führungsblock dafür einen Durchgangsschlitz auf, durch welchen eine sich hin- und herschwenkende, plane Schneide eines chirurgischen Instruments (Säge) hindurchgeführt wird.

Um die Ausrichtung der Tibia Resektionsebene einzustellen, wird nahe dem Fußknöchel des Patienten eine Fixierungsklammer bzw. Fixierungsklemme angebracht, welche an dem Einstellstab an seinem einen zu einem Fuß des Patienten hin weisenden Ende angebunden ist, an dessen anderem Ende (Endabschnitt) der Säge-/Führungsblock fixiert/fixierbar ist.

### Stand der Technik

Die US 6,221,035 B1 offenbart eine (Fixierungs-)Klammer für eine Ausrichtungshilfe, welche bei einer tibialen Resektion verwendet wird. Die Klammer weist zwei federvorgespannte Klammerarme auf, die gegenüber einem Rahmen um jeweils eine Drehachse gedreht werden können. Diese Klammerarme werden in eine geöffnete Position gebracht und nach Anlage an das Schienbein hiernach mittels einer manuellen Auslösevorrichtung freigegeben. Sie umschließen dann aufgrund der Federvorspannung ein Fußgelenk bzw. die Tibia und klemmen diese ein. Die Klammerarme sind dabei in die Schließ-Richtung vorgespannt. Die Federvorspannung bewirkt eine kraftschlüssige Fixierung, was jedoch zum Nachteil hat, dass beim Patienten an den betreffenden Körperstellen aufgrund der Klemmkraft Hämatome entstehen können. Die Klammerarme wirken federbelastend auf das Fußgelenk bzw. auf den Knöchel. Die Fixierungsklammer ist jedoch nicht an eine Anatomie des Patienten angepasst, sodass die Klammerarme je nach Fußform und Fußgelenksdicke eine unterschiedlich starke Anpresskraft ausüben. Hierdurch ergibt sich auch eine je nach Ausprägung des Fußgelenks abhängige Haltekraft der Fixierungsklammer.

Ein Problem des Standes der Technik ist, dass die Fixierung am Knöchel des Patienten meist kraftschlüssig erfolgt bzw. die Klammerarme kraftschlüssig vorgespannt werden, wodurch sich durch die dadurch entstehende Eigenelastizität der Klammerarme eine nur ungenügende Positionierbarkeit ergibt. Eine Anpassung an eine Anatomie des Patienten unterbleibt. Dies führt im schlechten Fall zu einer lösbaren Fixierung, die jedoch aufgrund der hohen Anforderungen an eine Maßhaltigkeit bzw. Genauigkeit an die eingangs beschriebene Ausrichtung der Ebene der Resektion nicht ausreichend ist. Auch können durch die unterschiedlichen Klemmkräfte elastisch vorgespannter Klemmarme Hämatome an den Körperstellen des Patienten entstehen. Durch die Fixierungsklammern nach dem Stand der Technik werden nicht alle anatomischen Größen abgedeckt, da die Klemmkraft abhängig von der jeweiligen Anatomie des Patienten ist. Daher müssten für eine optimale Anpassung verschiedene Varianten von Fixierungsklammern hergestellt und bereitgehalten werden, was jedoch in der Praxis aufgrund von hohen Kosten und schlechter Handhabbarkeit nicht praktiziert wird. Bei Fixierungsklammern, welche federbelastenden gegen das Fußgelenk bzw. den Knöchel anliegen besteht das Problem, dass je nach Fußform und Fußgelenksdicke eine unterschiedlich starke Anpresskraft durch die Feder ausgeübt wird.

### Zusammenfassung der Erfindung

Es ist daher die Aufgabe Erfindung, die Nachteile aus dem Stand der Technik zu vermeiden oder wenigstens zu mindern und insbesondere eine Fixierungsklammer sowie eine Ausrichtungsvorrichtung zur Verfügung zu stellen, die eine einfache, sichere und schnelle Fixierung sowie ein einfaches und schnelles Lösen der Fixierung von einer Körperextremität / eines Körpergliedes, insbesondere an bzw. um das Sprunggelenk bzw. um die Tibia, ermöglichen, wobei die Fixierungsklammer und die Ausrichtungsvorrichtung für unterschiedliche Anatomien von Körperextremitäten, insbesondere des Sprunggelenks angepasst sind und für jede Anatomie eingesetzt werden können und durch ihre Funktionsweisen und Konfigurationen Hämatome vermeiden. Auch soll die Fixierungsklammer eine möglichst einfache Konstruktion aufweisen, die vorzugsweise gut zu montieren, reinigen und sterilisieren ist.

Die Aufgabe wird hinsichtlich der Fixierungsklammer durch die Merkmale des Anspruchs 1 und hinsichtlich der Ausrichtungsvorrichtung durch die Merkmale des nebengeordneten Anspruchs 10 gelöst.

Der Kern der vorliegenden besteht demzufolge darin, dass an der Lagergabel als Grundkörper, die zwei unter einem Winkel zueinander angeordneten starren Lagerschenkel als Streben aufweist, die an den Drehgelenken angelenkten Klammerarme unabhängig voneinander an den entsprechenden Lagerschenkel werkzeuglos verschoben und positioniert werden können. Das heißt, dass die Fixierungsklammer gegenüber der Lagergabel auswählbar positionierbare Drehachsen für die Klammerarme aufweist.

Mit anderen Worten kann ein Abstand von der jeweiligen Drehachse der Klammerarme zu einem Fußpunkt der sich treffenden Lagerschenkel zwischen einem minimal möglichen Abstand und einem maximal möglichen Abstand kontinuierlich eingestellt und so an eine Anatomie individuell angepasst werden. Insbesondere kann das Drehgelenk mit dem entsprechend angelenkten Klammerarm entlang einer Längsachse des entsprechenden Lagerschenkels mittels einer Führung verlagert werden. Insbesondere stehen die Drehachsen (der Drehgelenke) senkrecht auf eine Längsachse der Lagerschenkel. Vorzugsweise sind die beiden Drehachsen der beiden Klammerarme parallel zueinander angeordnet. Alternativ können die Drehachsen auch einen gewissen Winkel zwischen sich aufweisen, insbesondere sich schneiden.

Sind beispielsweise die Klammerarme in eine Schließ-Drehrichtung federvorgespannt, so bleibt die Haltekraft der federvorgespannten Klammerarme auch bei Verschiebung der Drehgelenke gleich. Damit wird eine gleichbleibende Haltekraft der Fixierungsklammer unabhängig von der Anatomie des Fußes beispielsweise geschaffen.

Konkret ist also das erste Drehgelenk an dem ersten Lagerschenkel und/oder das zweite Drehgelenk an dem zweiten Lagerschenkel der Lagergabel verschiebbar gelagert.

Konstruktiv wird die erfindungsgemäße Idee dadurch umgesetzt, dass der erste und/oder zweite Lagerschenkel eine mechanische Führung oder ein Führungselement aufweist, in oder mit welcher das Drehgelenk verschiebbar geführt ist. Das Drehgelenk steht also in formschlüssigem Wirkeingriff mit der Führung der Lagerschenkel, wobei nur eine translatorische Verschiebung an dem Lagerschenkel zugelassen ist. Konkret weist also der Lagerschenkel eine Führungsfläche auf, auf welcher eine korrespondierende Gleitfläche des Drehgelenkt gleiten kann, wobei eine Korsettstruktur das Drehgelenk auf dem Lagerschenkel gleitend hält. Die Korsettstruktur kann dabei unterschiedlich ausgebildet sein. Beispielsweise kann die Korsettstruktur dadurch realisiert werden, dass das Drehgelenk in Richtung der Drehachse gesehen zwei sich gegenüberliegende plane Innenflächen aufweist, zwischen denen der Lagerschenkel eingefasst ist. So kann das Drehgelenk gleitend in einer Ebene relativ zu dem Lagerschenkel verschoben werden. Mithilfe der mechanischen Führung, beispielsweise in Form einer Führungsleiste ergibt sich durch die weitere geometrische Beschränkung aus der Ebene eine Kurve (Trajektionskurve). Auch kann die Korsettstruktur beispielsweise so ausgebildete sein, dass der Lagerschenkel zwei sich gegenüberliebende Innenflächen aufweist, zwischen denen das Drehgelenk eingefasst ist. Die Lagergabel der Fixierungsklammer weist also zum einen Gleitflächen auf, auf welcher die Drehgelenke abgleiten und translatorisch verschoben werden können und zum anderen Führungsflächen auf, die insbesondere senkrecht auf die Gleitflächen stehen, um das Drehgelenk auf der Gleitfläche entlang einer vorgegebenen Bahn zu führen.

Vorteilhafte Ausführungsformen sind in den Unteransprüchen beansprucht werden nachfolgend erläutert.

Gemäß einer bevorzugten Ausführungsform kann das erste Drehgelenk in Form eines Drehlagers an dem ersten Lagerschenkel und/oder das zweite Drehgelenk in Form eines Drehlagers an dem zweiten Lagerschenkel verschiebbar aber (im Wesentlichen) drehfest gelagert sein, so dass eine Ausrichtung des entsprechenden Drehlagers gegenüber dem entsprechenden Lagerschenkel auch bei einer Verschiebung beibehalten wird. Mit anderen Worten ist das Drehlager selbst, in dem der Klammerarm um die Drehachse drehbar gelagert ist, über eine Abstützfläche an sowohl dem Drehgelenk als auch dem Lagerschenkel gegen Verdrehen gesichert. Man kann auch sagen, dass das Drehgelenk im Querschnitt gesehen zumindest eine von einer kreissymmetrischen Form abweichende Kontur als Anlagefläche aufweisen muss, insbesondere eine Kontur mit zwei parallelen Kanten / Seiten, vorzugsweise eine im Wesentlichen rechteckförmige Kontur, die in Zusammenwirken mit einer korrespondierenden Abstützfläche des Lagerschenkels, deren eine Erstreckungsrichtung parallel zu der Drehachse liegt, ein Verdrehen verhindert. Dadurch kann eine Orientierung des Drehgelenks beibehalten werden.

Vorzugsweise kann das verschiebbar gelagerte erste Drehgelenk und/oder zweite Drehgelenk gegenüber der Lagergabel einen Selbsthemmmechanismus aufweisen, der das erste Drehgelenk bzw. das zweite Drehgelenk reib- und/oder formschlüssig in zumindest einer Position gegenüber der Lagergabel hält und eine Verschiebebewegung hemmt. Ein solcher Selbsthemmmechanismus kann in unterschiedlichen Formen ausgeführt sein. Beispielsweise kann das Drehgelenk elastisch vorgespannt den zugehörigen Lagerschenkel zwischen sich einpressen und gegen diesen drücken, so dass ein Reibwiderstand als Selbsthemmmechanismus das Drehgelenk in Position hält. Wird der Reibwiederstand, beispielsweise durch manuelle Betätigung aufgeboben, so wird der Selbsthemmmechanismus aufgehoben und das Drehgelenk kann wieder verschoben werden. Auch ist denkbar, dass beispielsweise eine Schraube die insbesondere in einem Gewinde des Drehgelenks eingeschraubt ist und dessen Ende an dem Lagerschenkel anliegt, auf den Lagerschenkel eine einstellbare Klemmkraft aufbringt und so eine Verschiebebewegung hemmt. Alternativ oder zusätzlich kann der Selbsthemmmechanismus auch formschlüssig ausgebildet sein, beispielsweise indem ein Verrastmechanismus zwischen dem Drehgelenk und dem zugehörigen Lagerschenkel ausgebildet ist.

Gemäß einer weiteren bevorzugten Ausführungsform kann der Selbsthemmmechanismus aktivierbar und deaktivierbar sein. Das heißt, dass der Selbsthemmmechanismus manuell eingeschaltet und ausgeschaltet werden kann und somit (zumindest) zwei unterschiedliche Zustände des Selbsthemmmechanismus vorliegen. In einem Fall von beispielsweise einem formschlüssigen Verrastmechanismus kann dieser manuell außer Wirkeingriff gebracht werden.

Insbesondere kann der Selbsthemmmechanismus bei einem auf das erste Drehgelenk aufgebrachten Drehmoment, insbesondere durch Übertragung des Drehmoments durch den ersten Klammerarm bzw. bei einem auf das zweite Drehgelenk aufgebrachten Drehmoment, insbesondere durch Übertragung des Drehmoments durch den zweiten Klammerarm, durch eine Verkantung zwischen dem Drehgelenk und dem Lagerschenkel aktiviert sein und bei Wegfall des Drehmoments deaktiviert sein. Ähnlich einer Einstellmöglichkeit einer Schraubzwinge, bei der ein Winkel durch Verdrehung zwischen zwei Flächen derart geändert wird, dass diese nicht mehr parallel, sondern einen Winkel zwischen sich aufweisen und dadurch verkanten, wird auch bei dem Selbsthemmmechanismus das Drehgelenk gegenüber dem zugehörigen Lagerschenkel (leicht) verdreht. Das Drehgelenk weist dabei vorzugsweise zwei (zumindest teilweise) parallel ausgebildete Leitflächen auf, die in dem Lagerschenkel eingefasst durch parallele Innenflächen geführt sind, wobei die Leitflächen nicht direkt an dem Lagerschenkel anliegen, sondern ein leichtes Spiel aufweisen. Wird nun das Drehgelenk in Folge eines aufgebrachten Drehmoments gedreht, so liegen in Folge die Leitflächen bzw. Kanten der Leitflächen direkt an den Innenflächen der Lagerschenkel an und es wird eine reibschlüssige Verbindung aufgebaut.

Vorzugsweise kann das erste Drehgelenk und/oder das zweite Drehgelenk über einen Führungsblock in einem in dem entsprechend ersten Lagerschenkel bzw. zweiten Lagerschenkel ausgebildeten Langloch verschiebbar geführt sein. Das Langloch, welches zwei parallele gegenüberliegende Innenflächen aufweist, umgreift dabei den Führungsblock des Drehgelenks, wobei der Führungsblock ebenfalls zwei parallel zueinander ausgebildete Leitflächen aufweist. Zwischen den Leitflächen und den Innenflächen ist ein kleiner Spalt ausgebildet bzw. es gibt ein Spiel, sodass auch noch eine Verdrehung der Leitflächen gegenüber den Innenflächen des Langlochs möglich ist. So kann der Führungsblock in dem Langloch solange geführt verschoben werden, solange die Leitflächen und die Innenflächen parallel zueinander liegen und bei Verdrehung des dreh-und axialfest mit dem Drehgelenk angebundenen Führungsblocks erfolgt eine Verkantung und der Selbsthemmmechanismus ist aktiviert und hemmt eine Verschiebebewegung des Drehgelenks in dem Lagerschenkel.

Es kann zweckdienlich sein, wenn der erste Klammerarm und/oder der zweite Klammerarm über eine Torsionsfeder, insbesondere über einer Drehfeder, in eine Schließ-Drehrichtung oder in eine Offen-Drehrichtung vorgespannt ist. Mittels der Drehfeder kann eine definierte Anpresskraft auf den Klammerarm übertragen werden. Die Drehfeder ist dafür zum einem an dem Drehgelenk, das drehfest an dem Lagerschenkel geführt ist, befestigt und zum anderen drückt sie gegen den Klammerarm und spannt diesen elastisch, insbesondere in die Schließ-Drehrichtung, vor. Damit kann ein Fuß federvorgespannt in der Fixierungsklammer mit definierter Federkraft eingeklemmt werden.

Gemäß einem weiteren Aspekt der Erfindung kann der erste Klammerarm und/oder der zweite Klammerarm über einen Dreh-Selbsthemmmechanismus, vorzugsweise in Form eines Verrastmechanismus gegenüber der Lagergabel in zumindest einer Position reib- und/oder formschlüssig gehalten oder verrastet sein und eine Bewegung des ersten bzw. zweiten Klammerarms um das jeweilige Drehgelenk in eine Offen-Drehrichtung und/oder eine Schließ-Drehrichtung sperren. Insbesondere kann der Verrastmechanismus, der den ersten Klammerarm und/oder den zweiten Klammerarm gegenüber einer Basis, als Lagergabel bezeichnet, in zumindest einer Position, vorzugsweise in einer Vielzahl von wählbaren Schwenkpositionen formschlüssig verrastet und eine Schwenk-Bewegung des ersten bzw. zweiten Klammerarms um das jeweilige Drehgelenk in eine Offen-Drehrichtung sperren. Die Offen-Drehrichtung ist dabei die Drehrichtung, in welche sich die (Spitzen der) Klammerarme voneinander wegbewegen um im Ergebnis die Fixierungsklammer zu öffnen. Mittels der formschlüssigen Verrastung und einer Sperrung der Offen-Drehrichtung der Klammerarme kann ein Anwender die Klemmung bzw. die geometrische Fixierung der Klemmarme und damit letztlich die Klemmkraft diskret einstellen. Es wird keine Klemmfeder mehr für eine kraftschlüssige Fixierung benötigt, sondern die Fixierung wird durch den Verrastmechanismus formschlüssig erreicht/gehalten. Der Anwender kann also die Klammerarme manuell (in die Schließ-Drehrichtung) zusammendrücken und sie in zumindest einer Position über den Verrastmechanismus arretieren. Liegen nun die Klammerarme um ein Sprunggelenk an diesem an, so können sie aufgrund ihrer Geometrie beispielsweise, in Zusammenwirken mit der Elastizität des Körpergewebes, leicht gegen dieses drücken, um einen festen Sitz zu erzielen. Eine Hämatombildung kann allerdings unterdrückt werden, da keine Federvorspannung aufgebracht wird, welche die Klammerarme über eine unkontrollierte, ggf. zu hohe Klemmkraft hinaus weiter in die Schließ-Drehrichtung zusammendrücken. Auch muss der Anwender die Klammerarme nicht entgegen der Vorspannkraft wieder auseinanderdrücken, sondern er kann, wenn der Formschluss auf eine bestimmte Art und Weise aufgehoben wird, die Klammerarme wieder in ihre ursprüngliche Position bewegen. Hierdurch wird eine Handhabung verbessert. Auch muss nicht sichergestellt werden, dass die Federkraft stets einem vorgegebenen Wert entspricht und insbesondere nicht über einen zeitlichen Rahmen hinweg ihre Vorspannung verliert. Damit wird auch eine Wartung vereinfacht oder entfällt.

In einer bevorzugten Ausführungsform kann der Dreh-Selbsthemmmechanismus in Form einer Ratschenmechanismus oder eines Freilaufs ausgeführt sein, um mittels Formschluss die Offen-Drehrichtung des zugehörigen Klammerarms zu sperren, wobei vorzugswiese über einen Betätigungskörper eine Sperrklinke des Ratschenmechanismus entgegen ihrer Vorspannung ausgelenkt wird und der Formschluss und damit der Wirkeingriff der Sperrklinke aufgehoben, so dass der zugehörige Klammerarm frei um seine Drehachse drehbar ist. Ein Ratschenmechanismus mit einem Sperrrad (Zahnrad) und einer (federvorgespannten) Sperrklinke ist eine besonders geeignete Ausführung, bei der eine spezielle geometrische Form der Zähne eine Drehbewegung in eine Richtung ermöglicht und in die entgegengesetzte Drehrichtung sperrt. Der Ratschenmechanismus ermöglicht bei der Fixierungsklammer eine Bewegung des entsprechenden Klammerarms in die Schließ-Drehrichtung und sperrt die entgegengesetzte Bewegung in die Offen-Drehrichtung. Ein solches System ist in der Funktion ähnlich zu einem Gesperr-Mechanismus, bei dem eine Sperrklinke federvorgespannt in ein Sperrrad eingreift. Der Betätigungskörper ermöglicht dem Anwender zusätzlich eine manuelle Freigabe bzw. Entsperrung. Hierfür kann der Betätigungskörper insbesondere entlang des entsprechenden Lagerschenkels translatorisch bewegt werden wobei eine Anlagefläche positionsabhängig die Sperrklinke entgegen ihrer Vorspannung auslenkt, der Ratschenmechanismus außer (formschlüssigen) Wirkeingriff kommt und damit deaktiviert wird. Wird der Betätigungskörper wieder losgelassen, so kehrt die Sperrklinke, insbesondere aufgrund einer Vorspannung, in ihren "normalen Zustand" des Wirkeingriffs zurück und der Verrastmechanismus in Form des Ratschenmechanismus ist hiernach wieder aktiviert.

Die Aufgabe der Erfindung wird bei einer gattungsgemäßen Ausrichtungsvorrichtung für eine tibiale Resektionsführung zur Verwendung bei der Vorbereitung eines Kniegelenks zur Implantation einer Prothese, mit einem Einstellstab, welcher gegenüber einer Tibia eines Patienten ausrichtbar ist, einer Führungsvorrichtung an einem Ende des Einstellstabes, die dafür angepasst ist, während einer Resektion der Tibia ein Werkzeug zu führen, und einer an einem gegenüberliegenden Ende des Einstellstabes oder zu dem anderen Ende des Einstellstabs hin angeordnete Fixierungsklammer, die die Tibia des Patienten umgreift und fixiert, um den Einstellstab in Bezug auf die Tibia zu fixieren, erfindungsgemäß dadurch gelöst, dass eine erfindungsgemäße Fixierungsklammer eingesetzt ist. Die Ausrichtungsvorrichtung lässt sich durch die erfindungsgemäße Fixierungsklammer schnell und einfach an einem Schienbein bzw. gegenüber einer Tibia des Patienten ausrichten und anordnen und ebenso einfach wieder lösen, um die Ausrichtungsvorrichtung umzupositionieren oder zu entfernen. Eine Handhabung wird deutlich vereinfacht und Hämatomen wird vorgebeugt.

### Kurzbeschreibung der Figuren

Die vorliegende Erfindung wird nachfolgend anhand bevorzugter Ausführungsformen unter Bezugnahme auf die begleitenden Figuren näher erläutert.
- Fig. 1: zeigt eine perspektivische Ansicht einer erfindungsgemäßen Fixierungsklammer gemäß einer ersten bevorzugten Ausführungsform,
- Fig. 2: zeigt eine Draufsicht auf die Fixierungsklammer aus Fig. 1,
- Fig. 3: zeigt eine Seitenansicht der Fixierungsklammer aus Fign 1 und 2,
- Fig. 4: zeigt eine Längsschnittansicht durch ein Drehgelenk der Fixierungsklammer aus den Fign 1 bis 3,
- Fig. 5: zeigt eine Schnittansicht durch eine Lagergabel der Fixierungsklammer aus Fign. 1 bis 4,
- Fig. 6: zeigt eine perspektivische Teilansicht der Klammerarme der Fixierungsklammer aus Fign 1 bis 5,
- Fig. 7: zeigt eine perspektivische Ansicht einer erfindungsgemäßen Fixierungsklammer gemäß einer weiteren, zweiten bevorzugten Ausführungsform,
- Fign 8 und 9: zeigen eine perspektivische Detailansicht der Lagerung der Klammerarme der Fixierungsklammer gemäß der zweiten Ausführungsform aus Fig. 7.

Die Figuren sind lediglich schematischer Natur und sollen nur dem Verständnis der Erfindung dienen. Gleiche Elemente sind mit denselben Bezugszeichen versehen. Die Merkmale der verschiedenen Ausführungsformen können untereinander ausgetauscht werden.

### Detaillierte Beschreibung bevorzugter Ausführungsformen

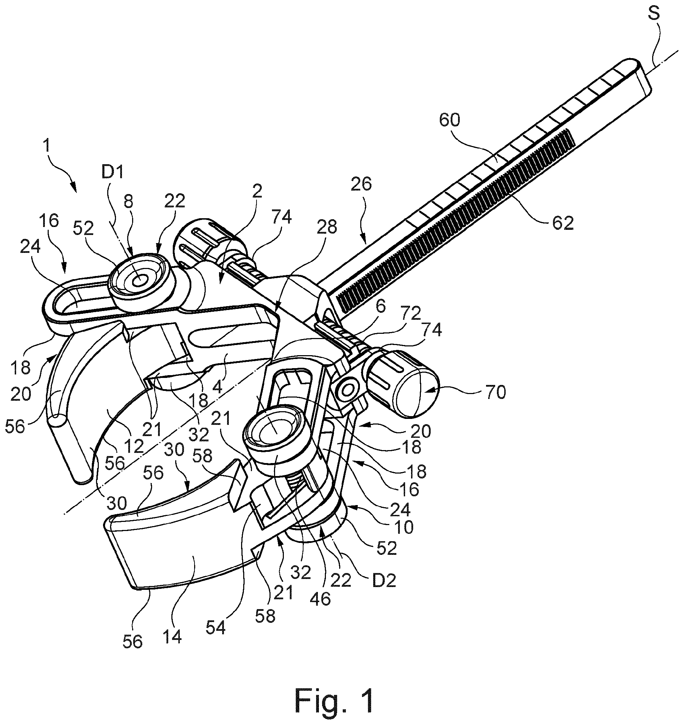

Figuren 1 bis 6 zeigen in einer perspektivischen Ansicht, in einer Draufsicht, in einer Seitenansicht, in einer Längsschnittansicht, in einer Schnittansicht und in einer perspektivischen Rückansicht eine erste bevorzugte Ausführungsform einer erfindungsgemäßen Fixierungsklammer 1 für eine Ausrichtungsvorrichtung (nicht dargestellt). Die Fixierungsklammer 1 weist eine (dreh-)symmetrisch zu einer Symmetrieachse S ausgebildete starre, Y-förmige Lagergabel 2 mit einem ersten Lagerschenkel 4 und einem zweiten Lagerschenkel 6 auf, die in Art einer Lagermulde ausgebildet ist, um eine Körperextremität eines Patienten geometrisch zwischen sich bzw. den beiden Lagerschenkel 4, 6 aufzunehmen und zu lagern. An dem ersten Lagerschenkel 4 ist mittelbar über ein erstes Drehgelenk 8 ein erster starr ausgebildeter Klammerarm (Klemmfinger) 12 und an dem zweiten Lagerschenkel 6 über ein zweites Drehgelenk 10 ein zweiter starr ausgebildeter Klammerarm (Klemmfinger) 14 jeweils um eine entsprechend erste Drehachse D1 bzw. eine zweite Drehachse D2 drehbar befestigt bzw. angelenkt, wobei erfindungsgemäß das erste Drehgelenk 8 zusammen mit dem ersten Klammerarm 12 und das zweite Drehgelenk 10 mit dem zweiten Klammerarm 14 an dem jeweiligen Lagerschenkel 4, 6 entlang seiner jeweiligen Längsachse verschoben und positioniert werden kann.

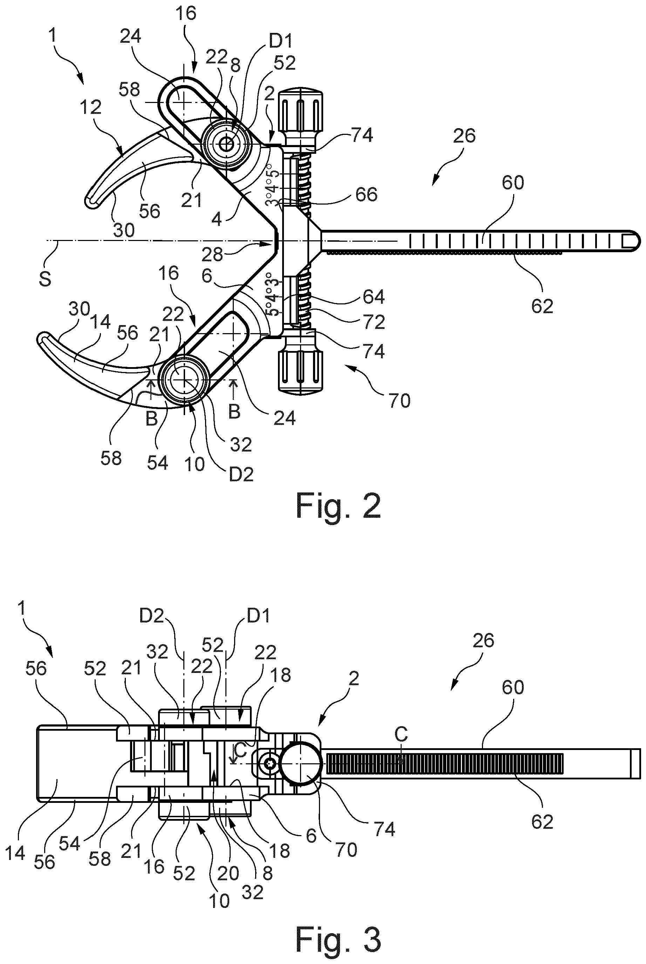

Dafür sind Endabschnitte 16 der beiden Lagerschenkel 4, 6 in einer Seitenansicht gesehen (siehe Fig. 3) jeweils gabel-/nutenförmig ausgebildet, bzw. weisen in Richtung der Drehachsen D1, D2 gesehen zwischen zwei planen, parallelen zu den Klammerarmen 12, 14 hin gewandten (Innen-)Flächen / Führungsflächen 18 der Lagerschenkel 4, 6 einen Spalt / eine Ausnehmung 20 auf. In diese Ausnehmung 20 der gabelförmigen Endabschnitten 16 ist der zugehörige Klammerarm 12, 14 mit planen (Außen-)Flächen / Führungsflächen 21 in der Art eines Scharniers eingefasst. Die Führungsflächen 18, 21 liegen plan aufeinander auf und erlauben eine Bewegung in einer Ebene. Ein Scharnierstift 22 steht dabei jeweils durch ein (in Fig. 3 gesehen) oberes Langloch 24 des Endabschnitts 16, durch den zugehörigen Klammerarm 12, 14, sowie durch ein weiteres, kongruent zu dem oberen Langloch 24 ausgebildet bzw. mit diesem fluchtend, (in Fig. 3 gesehen) unteres Langloch 24 hindurch. Der Scharnierstift 22 bildet also zusammen mit der scharnierartigen Einfassung des Klammerarms 12, 14 in die gabelförmigen Endabschnitte 16 der Lagerschenkel 4, 6 das jeweilige Drehgelenk 8, 10 aus, wobei sich aufgrund der Führung des jeweiligen Scharnierstifts 22 in den beiden zugehörigen Langlöchern 24 die Drehgelenke 8, 10 in Bezug auf die Lagergabel 2 translatorisch verschieben lassen.

Mit anderen Worten ausgedrückt sind die Endabschnitt 16 der Lagergabel 2 in Form eines Gabelgelenks ausgebildet, jedoch mit dem Unterschied, dass das Gabelgelenk nicht die zwei kreisförmigen fluchtenden Durchgangsbohrungen aufweist, sondern dass das Gabelgelenk stattdessen die zwei fluchtenden Langlöcher 24 aufweist, deren Breite dem Durchmesser des Scharnierstifts 22 entsprechen so dass der Scharnierstift 22 in den Langlöchern 24 in eine Längsrichtung der Lagerschenkel 4, 6 translatorisch führbar ist.

Nachfolgend wird die der Fixierungsklammer 1 immanente Funktion gemäß der ersten bevorzugten Ausführungsform erläutert. Konkret ist die Fixierungsklammer 1 als Baugruppe dafür vorbereitet in der Ausrichtungsvorrichtung eingesetzt zu werden und dort als geometrische Festlegung eines Fixierungspunktes der Ausrichtungsvorrichtung zu dienen. Um einen großen Abstand und einen langen Hebelarm für eine möglichst genaue Einstellung zu erreichen, wird die Fixierungsklammer nahe an dem Fußknöchel angebracht und mit einem Endabschnitt eines Einstellstabs, der zu dem Fuß des Patienten hinweist, der Ausrichtungsvorrichtung verbunden. Genauer gesagt ist die Lagergabel 2 mittelbar über einen starren Montage- oder Anbindungsabschnitt 26 an dem Einstellstab der Ausrichtungsvorrichtung (nicht dargestellt) lösbar befestigt und in die starre Lagergabel 2 wird der Fußknöchel ein- bzw. angelegt.

Erfindungsgemäß kann sowohl das erste Drehgelenk 8 translatorisch entlang des ersten Lagerschenkels 4 als auch (unabhängig hiervon) das zweite Drehgelenk 10 entlang des zweiten Lagerschenkels 6 bewegt werden, sodass eine Position der Drehgelenke 8, 10 an der Lagergabel 2 von einem Anwender wie einem Arzt eingestellt werden kann. Es kann also ein Abstand von dem ersten Drehgelenk 8 und von der ersten Drehachse D1 gegenüber einem Fußpunkt 28 der Y-förmigen Lagergabel 2, an dem die beiden Lagerschenkel 4, 6 sich treffen, innerhalb geometrisch vorgegebener Grenzen kontinuierlich eingestellt werden kann. Analog hierzu kann, unabhängig zu der Positionierung des ersten Drehgelenks 8, das zweite Drehgelenk 10 entlang des zweiten Lagerschenkels 6 bewegt und eingestellt werden.

In anderen Worten ausgedrückt ist also das erste Drehgelenk 8 an dem ersten Lagerschenkel 4 und das zweite Drehgelenk 10 an dem zweiten Lagerschenkel 6 der Lagergabel 2 verschiebbar gelagert. Durch diese beiden voneinander unabhängig einstellbaren Parameter des Abstandes der Drehachse D1, D2 zu dem Fußpunkt 28 der Lagergabel 2 kann die Fixierungsklammer 1 individuell an eine Anatomie eines Patienten angepasst werden. Auf diese Weise kann sichergestellt werden, dass die geometrisch vorbestimmten Klammerarme 12,14, welche eine konkave Anlagefläche 30 aufweisen, möglichst optimal an eine geometrische Form einer Malleole eines Schienbeins angepasst werden. Insbesondere können im Wesentlichen unterschiedliche Greif-Durchmesser der Fixierungsklammer 1 eingestellt werden. Beim Schließen der Klammerarme 12, 14 bildet sich mit der Lagergabel 2 und den Klammerarmen 12, 14 ein möglichst eng an dem anatomischen Umfang des Fußknöchels anliegender Rahmen. Dem Anwender werden also durch die Verlagerungsmöglichkeiten der Drehachsen D1, D2 an den entsprechenden Lagerschenkeln 4, 6 weitere geometrische Einstellmöglichkeiten geschaffen, die es nach dem Stand der Technik noch nicht gibt.

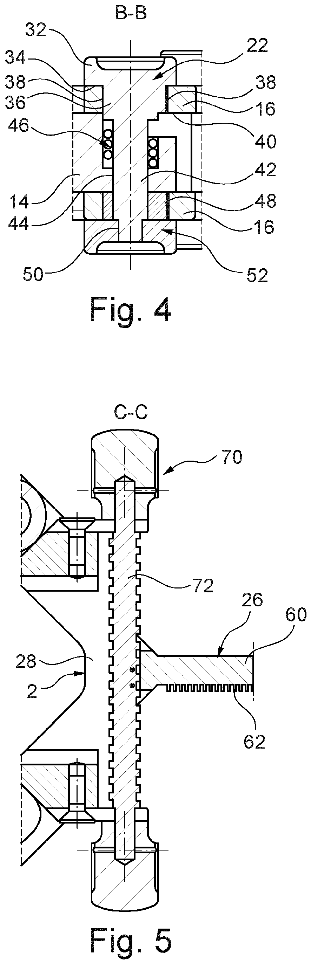

Nachstehend wird die Konfiguration der Drehgelenke 8, 10 unter Zuhilfenahme der Fig. 4, welche einen Längsschnitt B-B durch den Scharnierstift 22 zeigt, detailliert erläutert. Der Scharnierstift 22 weist entlang seiner Längsachse im Wesentlichen fünf unterschiedliche und direkt aufeinanderfolgende bzw. anschließende Abschnitte auf. Die Konstruktion und die Funktion werden anhand des zweiten Klammerarms 14 und des zweiten Lagerschenkels 6 erläutert, wobei der erste Klammerarm 12 und der erste Lagerschenkel 4 aufgrund der Punktsymmetrie zu der Symmetrieachse S entsprechend analog aufgebaut sind.

Konkret weist er als ersten Abschnitt (in Fig. 4 gesehen obersten Abschnitt) einen kreisrunden und drehsymmetrisch ausgebildeten Scharnierkopf 32 auf, dessen Durchmesser größer als die Breite des Langlochs 24 ist, um auf planen Auflageflächen 34 des Scharnierkopfs 32 auf den Endabschnitten 16 gleitend geführt zu werden und als Hinterschnitt den Scharnierstift 22 in Längsrichtung axial zu limitieren.

An dem Scharnierkopf 32 ist als zweiter Abschnitt unmittelbar folgend ein Führungsblock 36 ausgebildet. Dieser Führungsblock 36 weist zwei parallele, plane Leitflächen 38 auf, die sich der Längsachse des Scharnierstifts 22 gegenüberliegen. Der Abstand der beiden Leitflächen 38 ist geringer als der Durchmesser des Scharnierkopfs 32 und leicht geringer als die Breite des Langlochs 24. Konkret sind die beiden parallelen planen Leitflächen 38 von zwei gegenüberliegenden langen Innenflächen des Langlochs 24 eingefasst, wobei jedoch ein kleiner Spalt 40 bzw. ein kleiner Abstand zwischen den Leitflächen 38 und den beiden gegenüberliegenden Innenflächen des einen Langlochs 24 vorgesehen ist. Damit weist der Führungsblock 36 ein Spiel in Richtung quer zu der Längsachse der Lagerschenkel 4, 6 auf. Wird der Scharnierstift 22 in dem Langloch 24 gedreht, so sind aufgrund des Spiels die Leitflächen 38 des Scharnierstifts 22 und die Innenflächen des Langlochs 24 nicht mehr parallel zueinander, sondern weisen einen (kleinen) Winkel zwischen sich auf. Dies bewirkt, dass nun die äußeren und gegenüber der Längsachse sich diametral gegenüberliegenden Kanten der Leitflächen 38 an den beiden gegenüberliegenden Innenflächen des Langlochs 24 anliegen und verkanten. Aufgrund des Reibschlusses lässt sich das jeweilige Drehgelenk 8, 10 nicht mehr verschieben, wodurch der Selbsthemmmechanismus realisiert wird. Wird also der Scharnierstift 22 in dem Langloch (minimal) gedreht, beispielsweise indem ein Drehmoment auf den Scharnierstift 22 aufgebracht wird, so hemmt dies reibschlüssig eine Verschiebebewegung des Scharnierstifts 22 und damit der zugehörigen Drehgelenke 8, 10 in der Lagergabel 2. Zusätzlich können natürlich die Innenflächen der Langlöcher 24 und/oder die Leitflächen 38 des Scharnierstifts 22 so ausgebildet sein, dass neben einer reibschlüssigen Verbindung noch eine formschlüssige Verbindung tritt, welche eine Verkantung weiter unterstützt. Beispielsweise kann die jeweilige Fläche eine geriffelte Struktur aufweisen.

Als dritter Abschnitt schließt sich an den Führungsblock 36 in Längsachsenrichtung des Scharnierstifts 22 ein zylinderförmiger Wellenabschnitt 42 an, um welchen der jeweilige Klammerarm 12, 14 um die entsprechende Drehachse D1, D2 drehbar gelagert ist. Wie in Fig. 4 ersichtlich dient jedoch nicht der gesamte Wellenabschnitt 42 der Lagerung des entsprechenden Klammerarms 12, 14, sondern lediglich ein unterer Teilbereich des kreisförmigen Wellenabschnitts 42 wird von einer korrespondierenden kreisförmigen Öffnung 44 des Klammerarms 12, 14 umgriffen. Der restliche Bereich des Wellenabschnitts 42 wird hingegen von einer Drehfeder 46 umschlungen. Ein Endabschnitt der Drehfeder 46 ist einerseits an dem Führungsblock 36 fixiert und ein anderer Endabschnitt der Drehfeder 46 ist an dem Klammerarm 12, 14 derart angeordnet, dass die Drehfeder 46 diesen in eine Schließ-Drehrichtung (eine Drehrichtung, in welcher der Klammerarm 12 14 zu dem anderen Klammerarm 12, 14 hin bewegt wird; eine Drehbewegung zu der Symmetrieachse S hin) vorspannt.

Wird also auf den jeweiligen Klammerarm 12, 14 eine Kraft aufgebracht, beispielsweise dadurch, dass dieser an in eine Offen-Drehrichtung ausgelenkt wird, so übertragt die Drehfeder 46 diese Kraft auf den Führungsblock 36. Da die Kraft in einer Ebene senkrecht zu der Längsachse des Scharnierstifts 22 und versetzt zu dieser angreift, wird auf den Führungsblock 36 ein Drehmoment übertragen und dieser dreht sich gegenüber seinem Langloch 24 als Einfassung. Der Führungsblock 36 verkantet sich in dem Langloch und hemmt oder unterbindet eine Verschiebemöglichkeit des Drehgelenks 8, 10 entlang seines zugehörigen Lagerschenkels 4, 6.

Damit ist also folgende Funktion möglich: Der Anwender kann die Drehgelenke 8, 10 mit den Klammerarmen 12, 14 frei und werkzeuglos entlang der Lagerschenkel 4, 6 verschieben, bis er eine gewünschte Position erreicht, kann hiernach eine Kraft, insbesondere in die Offen-Drehrichtung, auf die Klammerarme 12, 14 aufbringen, also gegen diese drücken und hemmt oder unterbindet mit der Kraft so eine weitere Positionsänderung des Drehgelenks 8, 10. Das heißt, dass die Drehgelenke 8, 10 solange verschoben werden können, bis eine Belastung der Klammerarme 12, 14 um die Drehachsen D1, D2 auftritt. Je größer die Kraft ist, desto mehr verkanten sich die Führungsblöcke 36 in den Langlöchern 24 und hindern eine Verschiebebewegung. Unabhängig von der Position der Drehgelenke 8, 10 der Klammerarme 12, 14 bleibt eine Federkraft jedoch weiterhin gleich. Damit kann ein fester Halt der Fixierungsklammer 1 unabhängig von einer Fußform im Bereich des Fußgelenks des Patienten erreicht werden.

Der Anwender kann die Fixierungsklammer 1 an die Malleolen oder oberhalb oder unterhalb bzw. im Bereich der Malleolen anlegen, die Klammerarme 12, 14 werden durch die Fußform in die Offen-Drehrichtung entgegen der Vorspannung der Drehfeder 46 auseinandergedrückt, der Scharnierstift 22 verkantet aufgrund des von der Drehfeder 46 übertragenen Drehmoments und die Drehgelenke 8, 10 bleiben in ihrer Position gegenüber den Lagerschenkeln 4, 6.

Auf den Wellenabschnitt 42 folgt ein weiterer Führungsblock 48, der wieder, gleich zu dem Führungsblock 36, die planen parallelen Leitflächen 38, die in dem Langloch 24 geführt sind, und den Spalt 40 zwischen den Leitflächen 38 und den Innenflächen des Langlochs 24 aufweist. Der weitere Führungsblock 48 ist für eine gute Montage auf den Scharnierstift 22 aufgesteckt und drehfest mit diesem verbunden. Dieser weitere Führungsblock unterstützt das Verkanten und dient einer Lagerung des Scharnierstifts 22 in dem Langloch 24.

Als fünften und letzten Abschnitt (in Fig. 4 gesehen unterster Abschnitt) weist der Scharnierstift 22 ein Außengewinde 50 auf, welches in ein Innengewinde einer Mutter 52 eingreift, die einen gleichen Durchmesser wie der Scharnierkopf 32 aufweist. Damit lässt sich das Drehgelenk 8, 10 gut montieren, da die Durchmesser bzw. Abstände von dem ersten Abschnitt zu dem fünften Abschnitt hin stufenweise abnehmen. Somit kann der Scharnierstift 22 durch das in Fig. 4 gesehen obere Langloch 24, durch die Drehfeder 46, durch zugehörigen den Klammerarm 12, 14, durch das in Fig. 4 gesehen untere Langloch 24 und anschließend mit der Mutter 52 axial fixiert werden. Der Scharnierkopf 32 und die Mutter 52 weisen dabei in Längsrichtung eine Mulde bzw. Einbuchtung auf, um sie mit Daumen und Zeigefinger gut greifen und verschieben zu können

Wie in Fig. 1 beispielsweise ersichtlich sind die Klammerarme 12, 14 fingerartig geformt, wobei gegenüber der konkaven Anlagefläche 30 und im Bereich der Drehachse eine Einbuchtung 54 ausgebildet ist. In dieser Einbuchtung 54 liegt der eine Endabschnitt der Drehfeder 46 an. Zudem ist im Bereich der Spitzen der Klammerarme 12, 14, auf Höhe der beiden in dem Lagerschenkel 4, 6 ausgebildeten Langlöcher 24, eine Arm-Erweiterung 56 mit Anschlag 58 ausgebildet. Wie beispielsweise in Fig. 1 und 2 ersichtlich, begrenzt der Anschlag 58 einen Drehwinkel um die Drehachse D1, D2, wenn das Drehgelenk 8, 10 nahe dem Fußpunkt 28 angeordnet ist.

Die Führungsblöcke 36, 48 sind zu den Spitzen der Lagerschenkel 4, 6 hin mit einer halbkreisförmigen Kontur gestaltet, so dass sie, wenn das Drehgelenk 8, 10 in einer Position mit maximalmöglichem Abstand zu dem Fußpunkt 28 verschoben ist, in der korrespondierende halbkreisförmige Kontur des Langlochs 24 einliegen.

In Fig. 1 und 2 ist die Fixierungsklammer 1 in einem (teilgeöffneten) Zustand gezeigt. Die Klammerarme 12, 14 können in die Offen-Drehrichtung um ihre Drehachse D1, D2 gedreht und geöffnet werden und in diesem Zustand kann die Fixierungsklammer auf die Malleolen (oder darüber oder darunter) des Schienbeins des Patienten angeordnet werden, um diese später zu umgreifen / umfassen.

Die Fixierungsklammer 1 weist zur Anbindung/Befestigung/Montage an den Einstellstab einen blockförmigen, langgestreckten Kragarm / Kragträger 60 als Montage- oder Anbindungsabschnitt 26 auf. An einer Seitenfläche des Kragarms 60 ist eine Raststruktur 62 in Form einer Zahnstange ausgebildet. Der Kragarm 60 kann gegenüber dem Einstellstab längsverschoben werden, um eine Position in der Sagittalebene bzw. einen Versatz zwischen der Fixierungsklammer 1 und der Ausrichtungsvorrichtung einzustellen, und formschlüssig über die Raststruktur 62 fixiert werden.

Die Y-förmige Lagergabel 2 ist in der bevorzugten Ausführungsform zweiteilig ausgeführt, wobei die beiden Lagerschenkel 4, 6 als Gabelkopf den einen Teil und der Montage- oder Anbindungsabschnitt 26 den anderen Teil bilden.

Der Gabelkopf weist eine ebene Gleitfläche 64 an der zum Anbindungsabschnitt 26 hin zugewandten Seite auf, welche auf einer planen Gleitfläche 66 des Anbindungsabschnitts 26 translatorisch in eine Einstellrichtung gleiten kann. Um auf dem Anbindungsabschnitt 26 in Einstellrichtung nur axialbeweglich angebunden zu sein, weist der Gabelkopf zwei L-Förmige Führungsleisten bzw. Führungsvorsprünge 68 auf, die jeweils, ähnlich wie bei einer Führungsschiene, von Führungshaken / Umgreifungen 69 des Anbindungsabschnitts 26 eingefasst und umgriffen werden.

Wie in Fig. 5 gezeigt, weist der Gabelkopf ferner eine Drehspindel 70 als Dreheinstellung auf. Ein drehbarer Gewindestift bzw. eine drehbare Gewindespindel 72 mit Außengewinde greift in einen (Adapter)Block mit Innengewinde ein, der an dem Anbindungsabschnitt befestigt ist. Der Gewindestift 72 steht ferner durch ein Bohrloch eines Stegs 74 hindurch und ist durch diesen axialfest an dem Gabelkopf befestigt, so dass eine Drehung des Gewindestifts 72, entsprechend der Steigung des Außengewindes, eine translatorische Verschiebung des Blocks und damit des Gabelkopfs in die Einstellrichtung bewirkt. Damit kann die Ausrichtung des Gabelkopfs zu dem Anbindungsabschnitt 26 und damit auch zum Kragarm 60 eingestellt werden. Damit kann die Fixierungsklammer 1 der Ausrichtungsvorrichtung unter anderem eine Anpassung der Neigung einer Führungsvorrichtung und damit der Resektionsebene bewirken.

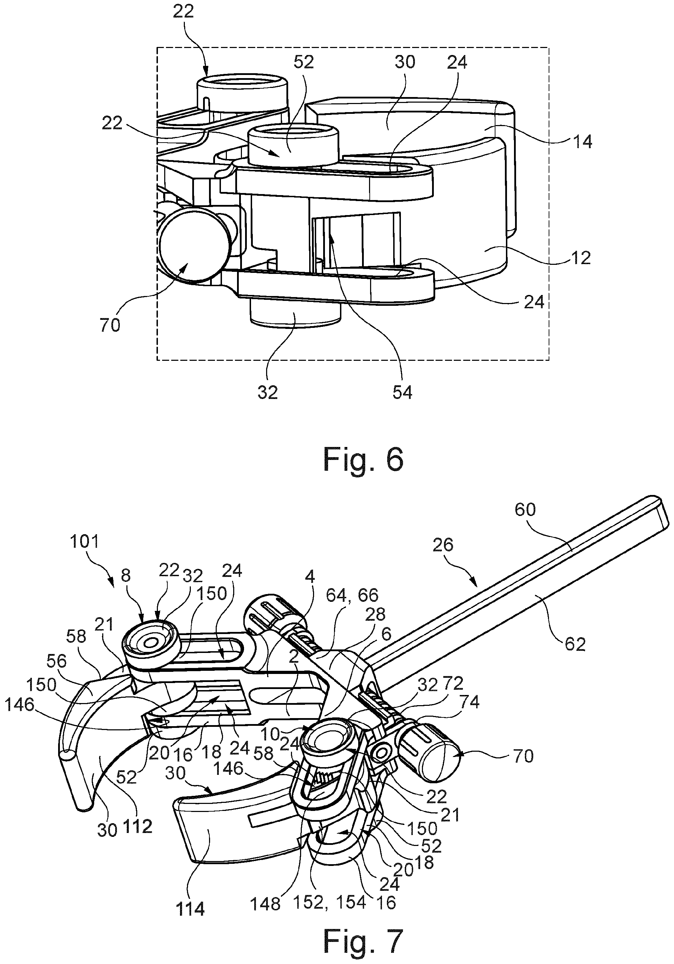

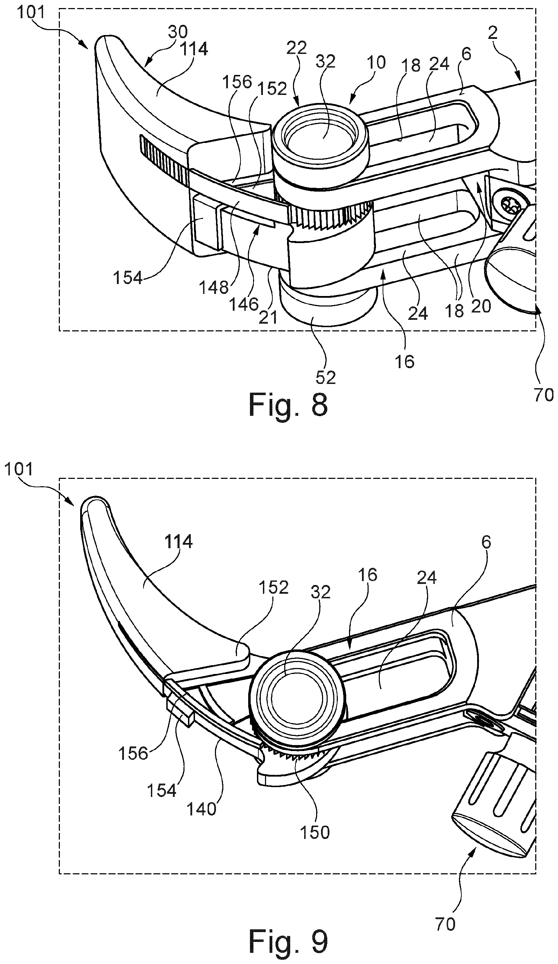

Fign. 7 bis 9 zeigt eine Fixierungsklammer 101 gemäß einer weiteren, zweiten bevorzugten Ausführungsform. Die Fixierungsklammer 101 entspricht im Wesentlichen der Fixierungsklammer 1 der ersten bevorzugten Ausführungsform mit dem Unterschied, dass statt der mittels der Drehfeder 46 kraftschlüssig klemmenden Klammerarme 12, 14 nun Klammerarme 112, 114 eingesetzt sind, welche nun einen formschlüssigen Verrastmechanismus ermöglichen. Genauer gesagt weist die Fixierungsklammer 1 einen Ratschenmechanismus 146 zum Halten der Klammerarme 112, 114 auf, wobei an dieser Stelle darauf hingewiesen wir, dass der Verrastmechanismus bzw. Haltemechanismus auch ein Reibschlussmechanismus sein kann.

Der Ratschenmechanismus 146 als Form eines Dreh-Selbsthemmmechanismus, sperrt den zugehörigen Klammerarm 112, 114 in die Offen-Drehrichtung, wobei jeweils eine an dem Klammerarm 112, 114 befestigte Sperrklinke 148 in eine Raststruktur in Form eines Zahnrads 150, das drehfest mit dem Scharnierstift 22 verbunden und damit mittelbar auch mit der Lagergabel 2 drehfest verbunden ist, eingreift. Die Sperrklinke 148 ist in Form eines Blechs ausgeführt, dessen Endabschnitt (Kante) vorgespannt in die Verzahnung des Zahnrads 150 drückt. Das Zahnrad 150 wiederum weist sägezahnförmige Vorsprünge mit asymmetrischen Zahnflanken auf. Damit wird durch die Sperrklinke 148 und dem Zahnrad 150 ein Gesperr gebildet, das einerseits ein aufeinander Abgleiten der Sperrklinke 148 auf dem Zahnrad 150 in die in die Schließ-Drehrichtung zulässt und andererseits verhindert, dass die Klammerarme 112, 114 in die Offen-Drehrichtung bewegt werden.

Über einen Betätigungshebel 152 in Form eines Exzenterhebels kann die Sperrklinke 148 entgegen ihrer Vorspannung ausgelenkt und außer Wirkeingriff gebracht werden, so dass der zugehörige Klammerarm frei um seine Drehachse drehbar ist. Konkret ist der Betätigungshebel 152 an dem jeweils zugehörigen Klammerarm 112, 114 um eine Drehachse drehbar gelagert. Ein Betätigungsabschnitt 154 liegt an eine Außenseite der des Klammerarms 112, 114 an und kann manuell zu dem zugehörigen Drehgelenk 8, 10 hingezogen werden. Der Betätigungshebel 152 dreht sich um die Drehachse, wobei ein an dem Betätigungshebel ausgebildeter Exzenterabschnitt 156 die Sperrklinke 148 aushebelt und außer Wirkeingriff bringt. Der Klammerarm 112, 114 kann frei in die Offen-Drehrichtung gedreht werden.

### Bezugszeichen

- 1; 101: Fixierungsklammer
- 2: Lagergabel
- 4: Erster Lagerschenkel
- 6: Zweiter Lagerschenkel
- 8: Erstes Drehgelenk
- 10: Zweites Drehgelenk
- 12; 112: Erster Klammerarm
- 14; 114: Zweiter Klammerarm
- 16: Endabschnitt
- 18: Innenfläche
- 20: Ausnehmung
- 21: Außenfläche
- 22: Scharnierstift
- 24: Langloch
- 26: Montage- oder Anbindungsabschnitt
- 28: Fußpunkt
- 30: Anlagefläche Klammerarm
- 32: Scharnierkopf
- 34: Auflagefläche
- 36: Führungsblock
- 38: Leitfläche
- 40: Spalt
- 42: Wellenabschnitt
- 44: Öffnung
- 46: Drehfeder
- 48: Führungsblock
- 50: Außengewinde
- 52: Mutter
- 54: Einbuchtung
- 56: Erweiterung
- 58: Anschlag
- 60: Kragarm
- 62: Raststruktur
- 64: Gleitfläche
- 66: Gleitfläche
- 68: Führungsvorsprung
- 69: Führungshaken
- 70: Drehspindel
- 72: Gewindestift
- 74: Steg
- 146: Ratschenmechanismus
- 148: Sperrklinke
- 150: Zahnrad
- 152: Betätigungshebel
- 154: Betätigungsabschnitt
- 156: Exzenterabschnitt

- D1: Erste Drehachse
- D2: Zweite Drehachse
- S: Symmetrieachse

## Patentansprüche

1. Fixierungsklammer (1; 101) für eine Ausrichtungsvorrichtung zum fixierenden Klemmen einer Körperextremität, mit einem ersten Klammerarm (12; 112) und einem zweiten Klammerarm (14; 114) und mit einer Lagergabel oder Lagermulde (2) für die Körperextremität mit einem ersten Lagerschenkel (4) und einem zweiten Lagerschenkel (6), welche einen zentralen Montage- oder Anbindungsabschnitt (26) zur Montage oder Anbindung an einen Einstellstab sowie ein erstes Drehgelenk (8) und ein zweites Drehgelenk (10) an der Lagergabel (2) aufweist, an denen jeweils der entsprechend erste Klammerarm (12; 112) um eine erste Drehachse (D1) und der zweite Klammerarm (14; 114) um eine zweite Drehachse (D2) drehbar angelenkt ist, **dadurch gekennzeichnet, dass** das erste Drehgelenk (8) an dem ersten Lagerschenkel (4) und/oder das zweite Drehgelenk (10) an dem zweiten Lagerschenkel (6) der Lagergabel (2) verschiebbar gelagert ist.

2. Fixierungsklammer (1; 101) nach Anspruch 1 **dadurch gekennzeichnet, dass** das erste Drehgelenk (8) in Form eines Drehlagers an dem ersten Lagerschenkel (4) und/oder das zweite Drehgelenk (10) in Form eines Drehlagers an dem zweiten Lagerschenkel (10) verschiebbar aber drehfest gelagert ist, so dass eine Ausrichtung des entsprechenden Drehlagers (8, 10) gegenüber dem entsprechenden Lagerschenkel (4, 6) auch bei einer Verschiebung beibehalten wird.

3. Fixierungsklammer (1; 101) nach Anspruch 1 oder 2, **dadurch gekennzeichnet, dass** das verschiebbar gelagerte erste Drehgelenk (8) und/oder zweite Drehgelenk (10) gegenüber der Lagergabel (2) einen Selbsthemmmechanismus aufweist, der das erste Drehgelenk (8) bzw. das zweite Drehgelenk (10) reib- und/oder formschlüssig in zumindest einer Position gegenüber der Lagergabel (2) hält und eine Verschiebebewegung hemmt.

4. Fixierungsklammer (1; 101) nach Anspruch 3, **dadurch gekennzeichnet, dass** der Selbsthemmmechanismus aktivierbar und deaktivierbar ist.

5. Fixierungsklammer (1; 101) nach einem der Ansprüche 3 oder 4, **dadurch gekennzeichnet, dass** der Selbsthemmmechanismus bei einem auf das erste Drehgelenk (8) aufgebrachten Drehmoment bzw. bei einem auf das zweite Drehgelenk (10) aufgebrachten Drehmoment um die Drehachse (D1, D2) durch eine Verkantung zwischen dem Drehgelenk (8,10) und dem zugehörigen Lagerschenkel (4, 6) aktiviert ist und bei Wegfall des Drehmoments deaktiviert ist.

6. Fixierungsklammer (1; 101) nach einem der vorangegangenen Ansprüche, **dadurch gekennzeichnet, dass** das erste Drehgelenk (8) und/oder das zweite Drehgelenk (10) über einen Führungsblock (36, 48) in einem in dem entsprechend ersten Lagerschenkel (4) bzw. zweiten Lagerschenkel (6) ausgebildeten Langloch (24) verschiebbar geführt ist.

7. Fixierungsklammer (1) nach einem der vorangegangenen Ansprüche, **dadurch gekennzeichnet, dass** der erste Klammerarm (12) und/oder der zweite Klammerarm (14) über eine Torsionsfeder, insbesondere einer Drehfeder (46), in eine Schließ-Drehrichtung oder in eine Offen-Drehrichtung vorgespannt ist.

8. Fixierungsklammer (101) nach einem der vorangegangenen Ansprüche, **dadurch gekennzeichnet, dass** der erste Klammerarm (112) und/oder der zweite Klammerarm (114) über einen Dreh-Selbsthemmmechanismus, vorzugsweise in Form eines Verrastmechanismus (146), gegenüber der Lagergabel (2) in zumindest einer Position reib- und/oder formschlüssig hält oder verrastet und eine Bewegung des ersten bzw. zweiten Klammerarms (114, 112) um das jeweilige Drehgelenk in eine Offen-Drehrichtung und/oder eine Schließ-Drehrichtung sperrt.

9. Fixierungsklammer (101) nach Anspruch 8, **dadurch gekennzeichnet, dass** der Dreh-Selbsthemmmechanismus in Form eines Ratschenmechanismus (146) oder eines Freilaufs ausgeführt ist, um mittels Formschluss die Offen-Drehrichtung des zugehörigen Klammerarms (112, 114) zu sperren, wobei vorzugswiese über einen Betätigungshebel (152) eine Sperrklinke (148) des Ratschenmechanismus (146) entgegen ihrer Vorspannung ausgelenkt und der Formschluss und damit der Wirkeingriff der Sperrklinke (146) aufgehoben wird, so dass der zugehörige Klammerarm (112, 114) frei um seine Drehachse (D1, D2) drehbar ist.

10. Ausrichtungsvorrichtung für eine tibiale Resektionsführung mit:
einem Einstellstab, welcher gegenüber einer Tibia eines Patienten ausrichtbar ist,
eine Führungsvorrichtung an einem Ende des Einstellstabes, die dafür angepasst ist, während einer Resektion der Tibia ein Werkzeug zu führen, und
eine an einem gegenüberliegenden Ende des Einstellstabes angeordnete Fixierungsklammer, die die Tibia des Patienten umgreift und fixiert, um den Einstellstab in Bezug auf die Tibia zu fixieren
**dadurch gekennzeichnet, dass**
die Fixierungsklammer (1; 101) die Merkmale nach einem der Ansprüche 1 bis 9 aufweist.

## Claims

1. A fixing clamp (1; 101) for an aligning device for fixingly clamping a body extremity, comprising a first clamp arm (12; 112) and a second clamp arm (14; 114) and comprising a support fork or support trough (2) for the body extremity having a first support leg (4) and a second support leg (6), which has a central mounting or connecting portion (26) for mounting or connection to an adjusting rod as well as a first rotation joint (8) and a second rotation joint (10) on the support fork (2), on each of which the corresponding first clamp arm (12; 112) is rotatably articulated about a first rotational axis (D1) and the second clamp arm (14; 114) is rotatably articulated about a second rotational axis (D2), **characterized in that** the first rotation joint (8) is displaceably mounted on the first support leg (4) and/or the second rotation joint (10) is displaceably mounted on the second support leg (6) of the support fork (2).

2. The fixing clamp (1; 101) according to claim 1 **characterized in that** the first rotation joint (8) in the form of a pivot bearing is mounted on the first support leg (4) displaceably but rotationally fixed and/or the second rotation joint (10) in the form of a pivot bearing is mounted on the second support leg (10) displaceably but rotationally fixed, so that an orientation of the corresponding pivot bearing (8, 10) with respect to the corresponding support leg (4, 6) is maintained even during a displacement.

3. The fixing clamp (1; 101) according to claim 1 or 2, **characterized in that** the displaceably mounted first rotation joint (8) and/or second rotation joint (10) has a self-retaining mechanism relative to the support fork (2), which holds the first rotation joint (8) respectively the second rotation joint (10) frictionally and/or form-fittingly in at least one position relative to the support fork (2) and inhibits a displacement movement.

4. The fixing clamp (1; 101) according to claim 3, **characterized in that** the self-retaining mechanism is adapted to be activated and deactivated.

5. The fixing clamp (1; 101) according to one of claims 3 or 4, **characterized in that** the self-retaining mechanism is activated by a torque applied to the first rotation joint (8) respectively by a torque applied to the second rotation joint (10) about the rotational axis (D1, D2) by tilting between the rotation joint (8, 10) and the associated support leg (4, 6) and is deactivated when the torque is removed.

6. The fixing clamp (1; 101) according to one of the preceding claims, **characterized in that** the first rotation joint (8) and/or the second rotation joint (10) is displaceably guided via a guide block (36, 48) in an elongated hole (24) formed in the corresponding first support leg (4) respectively second support leg (6).

7. The fixing clamp (1) according to one of the preceding claims, **characterized in that** the first clamp arm (12) and/or the second clamp arm (14) is preloaded via a torsion spring, in particular a coil spring (46), in a closing rotational direction or in an opening rotational direction.

8. The fixing clamp (101) according to one of the preceding claims, **characterized in that** the first clamp arm (112) and/or the second clamp arm (114) frictionally and/or form-fittingly holds or locks in at least one position relative to the support fork (2) via a rotary self-retaining mechanism, preferably in the form of a latching mechanism (146), and blocks a movement of the first or of the second clamp arm (114, 112) about the respective rotation joint in an opening rotational direction and/or a closing rotational direction.

9. The fixing clamp (101) according to claim 8, **characterized in that** the rotary self-retaining mechanism is configured in the form of a ratchet mechanism (146) or a freewheel in order to lock the opening rotational direction of the associated clamp arm (112, 114) via form fit, wherein preferably a detent (148) of the ratchet mechanism (146) is deflected against its preload via an actuation lever (152), and the form fit and thus the operative engagement of the detent (146) is released, so that the associated clamp arm (112, 114) is freely rotatable about its rotational axis (D1, D2).

10. An aligning device for a tibial resection guide comprising:
an adjusting rod which is alignable with respect to a tibia of a patient,
a guiding device at one end of the adjusting rod adapted to guide a tool during a resection of the tibia, and
a fixing clamp arranged at an opposite end of the adjusting rod, which grips and fixes the patient's tibia in order to fix the adjusting rod in relation to the tibia
**characterized in that**
the fixing clamp (1; 101) comprises the features according to one of claims 1 to 9.

## Revendications

1. Pince de fixation (1; 101) destinée à un dispositif d'alignement de pinces à fixer d'une extrémité de corps, munie d'un premier bras de pince (12 ; 112) et d'un second bras de pince (14 ; 114) et munie d'une fourche de palier ou d'une auge de stockage (2) pour l'extrémité de corps avec une première branche de palier (4) et une seconde branche de palier (6), laquelle présente une section de montage ou de liaison centrale (26) pour le montage ou la liaison au niveau d'une tige de réglage, ainsi qu'une première articulation pivotante (8) et une seconde articulation pivotante (10) au niveau de la fourche de palier (2), au niveau desquelles respectivement le premier bras de pince (12 ; 112) correspondant est articulé en rotation autour d'un premier axe de rotation (D1) et le seconde bras de pince (14 ; 114) est articulé en rotation autour d'un second axe de rotation (D2),
**caractérisée en ce que** la première articulation pivotante (8) est montée de manière coulissante sur la première branche de palier (4) et/ou la seconde articulation pivotante (10) est montée de manière coulissante sur la seconde branche de palier (6) de la fourche de palier (2).

2. Pince de fixation (1; 101) selon la revendication 1 **caractérisée en ce que** la première articulation pivotante (8) sous la forme d'un palier de pivotement est montée de manière coulissante, mais solidairement en rotation, au niveau de la première branche de palier (4) et/ou la seconde articulation pivotante (10) sous la forme d'un palier de pivotement est montée de manière coulissante, mais solidairement en rotation, au niveau de la seconde articulation pivotante (10), de sorte qu'un alignement du palier de pivotement (8, 10) correspondant est maintenue par rapport à la branche de palier (4, 6) correspondante également lors d'un coulissement.

3. Pince de fixation (1 ; 101) selon la revendication 1 ou 2, **caractérisée en ce que** la première articulation pivotante (8) et/ou la seconde articulation pivotante (10) montée de manière coulissante par rapport à la fourche de palier (2) présente un mécanisme de verrouillage automatique, qui maintient la première articulation pivotante (8) ou la seconde articulation pivotante (10) par frottement et/ou par complémentarité de formes au moins dans une position par rapport à la fourche de palier (2) et empêche un mouvement de coulissement.

4. Pince de fixation (1; 101) selon la revendication 3, **caractérisée en ce que** le mécanisme de verrouillage automatique peut être activé et désactivé.

5. Pince de fixation (1; 101) selon l'une quelconque des revendications 3 ou 4, **caractérisée en ce que** le mécanisme de verrouillage automatique est activé lors d'un couple de rotation placé sur la première articulation pivotante (8) ou lors d'un couple de rotation placé sur la seconde articulation pivotante (10) autour d'un axe de rotation (D1, D2) par une inclinaison entre l'articulation pivotante (8, 10) et la branche de palier (4, 6) associée et est désactivé en cas de suppression du couple de rotation.

6. Pince de fixation (1 ; 101) selon l'une quelconque des revendications précédentes, **caractérisée en ce que** la première articulation pivotante (8) et/ou la seconde articulation pivotante (10) est dirigée de manière coulissante par le biais d'un bloc de guidage (36, 48) dans un trou oblong (24) formé dans la première branche de palier (4) ou la seconde branche de palier (6).

7. Pince de fixation (1) selon l'une quelconque des revendications précédentes, **caractérisée en ce que** le premier bras de pince (12) et/ou le second bras de pince (14) est précontraint par le biais d'un ressort de torsion, en particulier d'un ressort de rotation (46), dans une direction de rotation fermée ou dans une direction de rotation ouverte.

8. Pince de fixation (101) selon l'une quelconque des revendications précédentes, **caractérisée en ce que** le premier bras de pince (112) et/ou le second bras de pince (114) est maintenu ou encliqueté par le biais d'un mécanisme de verrouillage automatique de rotation, de préférence sous la forme d'un mécanisme d'encliquetage (146) par frottement et/ou par complémentarité de formes par rapport à la fourche de palier (2) dans au moins une position et bloque un mouvement du premier ou du second bras de pince (114, 112) autour de l'articulation pivotante respective dans une direction de rotation ouverte et/ou une direction de rotation fermée.

9. Pince de fixation (101) selon la revendication 8, **caractérisée en ce que** le mécanisme de verrouillage automatique de rotation est réalisé sous la forme d'un mécanisme à déclic (146) ou d'une roue libre, pour bloquer au moyen d'une complémentarité de formes la direction de rotation ouverte du bras de pince (112, 114) associé, dans lequel de préférence par le biais d'un levier de manoeuvre (152) un cliquet (148) du mécanisme à déclic (146) est dévié vers sa précontrainte et la complémentarité de formes et donc l'engagement actif du cliquet (146) est supprimé de sorte que le bras de pince (112, 114) associé peut pivoter librement autour de son axe de rotation (D1, D2).

10. Dispositif d'alignement destiné à un guide de résection tibiale comprenant :
une tige de réglage, laquelle peut être alignée par rapport au tibia d'un patient,
un dispositif de guidage au niveau d'une extrémité de la tige de réglage, qui est adapté pour guider un instrument pendant une résection du tibia, et
une pince de fixation disposée au niveau d'une extrémité opposée de la tige de réglage, qui entoure et fixe le tibia du patient, pour fixer la tige de réglage par rapport au tibia
**caractérisé en ce que**
la pince de fixation (1; 101) présente les caractéristiques selon l'une quelconque des revendications 1 à 9.
